# EUROPÄISCHE PATENTSCHRIFT

(11) **EP 1 713 387 B1**
(45) Veröffentlichungstag und Bekanntmachung des Hinweises auf die Patenterteilung: **02.04.2014**
(21) Anmeldenummer: 05707298.5
(22) Anmeldetag: 10.02.2005
(51) Int. Cl.: A61B 5/107, A61B 19/00, G02B 23/24, A61B 1/04

(54) **VERFAHREN UND VORRICHTUNG ZUM ERSTELLEN ZUMINDEST EINES AUSSCHNITTS EINES VIRTUELLEN 3D-MODELLS EINES KÖRPERINNENRAUMS**
METHOD AND DEVICE FOR CREATING AT LEAST ONE SECTION OF A VIRTUAL 3D MODEL OF A BODY CAVITY
PROCEDE ET DISPOSITIF DE CREATION D'AU MOINS UNE EXTRAIT D'UN MODELE 3D VIRTUEL D'UNE CAVITE NATURELLE DU CORPS

(30) Priorität: 11.02.2004 DE 102004008164
(43) Veröffentlichungstag der Anmeldung: 25.10.2006
(73) Patentinhaber: Karl Storz GmbH & Co. KG, 78532 Tuttlingen (DE); Ludwig-Maximilians-Universität, 80539 München (DE)
(72) Erfinder: DRAXINGER, Wolfgang, 80637 München (DE); NOLL, Margarita, 82234 Wessling (DE); STEPP, Herbert, 82152 Planegg (DE); IRION, Klaus, M., 78576 Emmingen-Liptingen (DE)
(74) Vertreter: Heuckeroth, Volker
(86) Internationale Anmeldenummer: PCT/EP2005/001316
(87) Internationale Veröffentlichungsnummer: WO 2005/077272

(56) Entgegenhaltungen:
- WO-A-01/35849
- DE-A1- 10 104 483
- DE-A1- 19 511 978
- DE-A1- 19 750 698
- DE-A1- 19 800 765
- US-A- 4 986 262
- US-A1- 2003 164 952

## Beschreibung

Die Erfindung betrifft ein Verfahren und eine Vorrichtung zum Erstellen zumindest eines Ausschnitts eines virtuellen 3D-Modells eines Körperinnenraums, insbesondere eines Hohlorgans.

Ein solches Verfahren und eine solche Vorrichtung werden allgemein bei der endoskopischen Untersuchung von Körperinnenräumen im menschlichen oder tierischen Körper angewandt bzw. verwendet. Ein solcher Körperinnenraum kann beispielsweise die Harnblase, die Lunge, der Magen, die Kehle, die Speiseröhre, usw. sein.

Ohne Beschränkung der Allgemeinheit wird die vorliegende Erfindung anhand des speziellen Anwendungsfalls der Untersuchung der Harnblase beschrieben.

Bei der endoskopischen Untersuchung der Harnblase versucht der untersuchende Arzt, das Organ möglichst flächendeckend mit dem Endoskop zu erfassen, das heißt "abzuscannen". Dabei ist es von Wichtigkeit, dass jeder Teil der Oberfläche der Harnblase überhaupt bzw. mit der ausreichenden Sorgfalt erfasst wird. Ob jeder Teil der Oberfläche der Harnblase erfasst wird, ist im Wesentlichen von der Erfahrung des Arztes abhängig. Es kann daher vorkommen, dass Bereiche der Wand der Harnblase übersehen werden, das heißt nicht endoskopisch erfasst werden. Handelt es sich bei den nicht erfassten Bereichen der Wand der Harnblase um ggf. maligne Gewebeareale, kann dies für den Patienten schwerwiegende Folgen haben.

Eine Ursache dafür, dass es bei der endoskopischen Untersuchung der Harnblase zu Auslassungen von Gewebearealen bzw. Oberflächenarealen der Harnblase kommt, ist die durch das eingeschränkte Blickfeld eines Endoskops erschwerte Orientierung des Arztes in dem im Wesentlichen kugelförmigen Innenraum der Harnblase. Das eingeschränkte Blickfeld eines Endoskops hat nämlich zur Folge, dass mit ein und demselben Endoskopiebild nicht die gesamte Oberfläche des Körperinnenraums erfasst wird, sondern nur ein bestimmtes Oberflächenareal.

Da das auf einem Monitor visuell dargestellte Endoskopiebild außerdem keine räumliche Tiefe besitzt, sondern lediglich "zweidimensional" wirkt, lässt sich allein anhand des erfassten Endoskopiebildes nicht beurteilen, von welchem Oberflächenareal dieses Endoskopiebild stammt. Werden beispielsweise mehrere Endoskopiebilder von unterschiedlichen Oberflächenarealen des Körperinnenraums erfasst und dokumentiert, und wird auf einem der Endoskopiebilder ein pathologischer Befund registriert, lässt sich später anhand der Endoskopiebilder nicht mehr eindeutig bestimmen, von welchem Oberflächenareal dieses Endoskopiebild mit dem pathologischen Befund stammt.

Somit ist auch das Wiederauffinden von in vorangegangenen Untersuchungen behandelten Gewebebereichen bei wiederholter endoskopischer Untersuchung, zum Beispiel bei der Kontrolle des Therapieverlaufs oder der Tumornachsorge, schwierig. Auch hier ist der Arzt von der Qualität der Befundsdokumentation abhängig. Aufgrund der großen Ähnlichkeit des Erscheinungsbilds verschiedener Oberflächenbereiche der Innenwand der Harnblase auf dem jeweiligen Endoskopiebild ist daher ein sicheres und rasches Wiederauffiriden eines bestimmten Oberflächenareals des Körperinnenraumes mit ausreichender Sicherheit nicht oder nur bedingt möglich.

Es besteht daher ein Bedürfnis an einer Bilderfassung und -dokumentation eines Körperinnenraumes, die eine verbesserte Orientierung des Arztes bei der Untersuchung des Körperinnenraums und vor allem eine Möglichkeit bieten, dass der Arzt feststellen kann, welche Oberflächenareale bereits untersucht und welche noch nicht untersucht wurden. Mit anderen Worten ist es wünschenswert, wenn dem untersuchenden Arzt ein Mittel zur Navigation des Endoskops im Körperinnenraum an die Hand gegeben wird, so dass er das Endoskop im Körperinnenraum gezielt so bewegen kann, dass die Oberfläche des Körperinnenraums möglichst rasch und vollständig endoskopiert erfasst und dokumentiert werden kann.

Im Stand der Technik sind bereits Verfahren und Vorrichtungen beschrieben worden, so zum Beispiel in dem Dokument DE 197 50 698 A1, mit denen ein Körperinnenraum dreidimensional vermessen werden kann, um ein virtuelles Modell, das heißt ein Computermodell, von dem Körperinnenraum zu erstellen. In dem genannten Dokument wird hierzu eine Aufsteckeinheit für ein flexibles Endoskop beschrieben, in die eine Sonde einführbar ist, die ein geordnetes Lichtleitfaserbündel mit einem Mantel beinhaltet. Der Mantel ist mit äquidistanten, durch berührungslose Abtaste- bzw. Auslesemittel erfassbaren Markierungen versehen. Dem Lichtleitfaserbündel ist ein optisches Abbildmittel zugeordnet, wobei dem Lichtleitfaserende ein Strahlablenkmittel zugeordnet ist, das eine rechtwinklige allseitige Lichtstrahlablenkung auf die Oberfläche des vermessenen Körperinnenraums ermöglicht.

Das Dokument US 5,704,897 beschreibt eine Vorrichtung und ein Verfahren, das eine Überlagerung einer optischen Darstellung eines Endoskopiebildes mit einem Datenfeld ermöglicht, um die Navigation eines Instruments während endoskopischer Operationen zu unterstützen. Ein Endoskopiebild wird mit einem Endoskop erfasst und auf einem Bildschirm dargestellt. An dem Endoskop ist ein Lageerfassungssystem angeordnet, um permanent die räumliche Position des Endoskops zu erfassen. Das räumliche Datenfeld, das ein Modell des Körperinnenraums bildet, wird durch Computertomografie, Magnetresonanztomografie oder Ultraschall erhalten und einem menschlichen Körper in einer bestimmten Lage zugeordnet. Ein Sensor, der an dem Körper des Patienten befestigt werden kann, dient zur Kompensation von Bewegungen des Körpers. Mittels eines Computers werden ausgewählte Punkte des Datenfeldes mit entsprechendes Punkten der optischen Darstellung durch Verschieben bestimmter Punkte des Datenfeldes, während diese der optischen Darstellung überlagert sind, in Übereinstimmung gebracht.

Ein weiteres Dokument, das sich mit der Dokumentation von Behandlungsverläufen beschäftigt, ist das Dokument DE 297 23 333 U1, das ein System zum Wiederauffinden bestimmter Stellen in einem Körperhohlraum offenbart. Dieses System weist einen Tubus zum Einführen in die Körperhöhle und ein Endoskop zum optischen Darstellen der den Tubus umgebenden Gewebeflächen auf, wobei der Tubus mindestens abschnittsweise durchsichtig ausgebildet ist und in seinen durchsichtigen Abschnitten optische Markierungen aufwest, die relativ zum Patienten reproduzierbar ausrichtbar sind, so dass bestimmte Bereiche der Gewebeoberfläche bestimmten Markierungen zuzuordnen und die bestimmten Bereiche anhand der Zuordnung wieder auffindbar sind.

Aus WO 01/35849 A1 ist eine Videoendoskopie-Vorrichtung für die computergestützte Chirurgie bekannt, die ein Triangulations-Laser-Profilometer aufweist. Ein Laserstrahl, der mit der optischen Achse des Video-Endoskops koplanar ist, wird zu einer Linie aufgespreizt. Die durch die Video-Endoskop-Kamera gesehene Verformung der Laserlinie ermöglicht es, den Abstand der Punkte auf der Laserlinie relativ zum Video-Endoskop zu ermitteln. Das Video-Endoskop wird durch Sensoren positioniert, wodurch die Position der Punkte der Laserlinie sowohl in einem zuvor gewonnenen Modell als auch in dem Video-Endoskopbild bekannt ist. Mit dieser bekannten Vorrichtung kann das videoendoskopische Bild mit dem zuvor mittels eines anderen Verfahrens gewonnenen Modells des Beobachtungsraums in Deckung gebracht werden, und es können Informationen aus dem zuvor gewonnenen Modell dem Video-Bild überlagert werden. Ebenso soll es möglich sein, das zuvor gewonnene Modell durch das Videoendoskopbild zu aktualisieren. Bei dem mit dieser bekannten Vorrichtung angewandten Verfahren kann die durch das Videoendoskop gesehene Verformung der Laserlinie dazu benutzt werden, für jedes Videobild den Abstand und die Orientierung jedes Punktes der Laserlinie bezüglich dem Mittelpunkt des Endoskopkopfes zu berechnen.

DE 198 00 765 A1 offenbart ein Verfahren zur Erzeugung von Bilddarstellungen der Oberfläche der Innenwand von Hohlkörpern im Rahmen einer endoskopischen Untersuchung, insbesondere von Hohlraumorganen und Gefäßen, wobei die mittels eines Endoskops gelieferten Bilddaten in Form von Videobildsignalen ausgegeben werden. Dabei werden Einzelbilder einander überlagert und/oder zusammengesetzt, und zwar durch Korrelation mittels Korrelationsalgorithmen und/oder aufgrund während der Einzelbildaufnahme erfasster räumlicher Positions- und/oder Orientierungsdaten. Das System umfasst ein Positions- und/oder Orientierungserfassungssystem zur Ermittlung der Positions- und/oder Orientierungsdaten. DE 198 00 765 A1 offenbart ein Verfahren entsprechend dem Oberbegriff des Anspruchs 1 und eine Vorrichtung entsprechend dem Oberbegriff des Anspruchs 12.

DE 101 04 483 A1 offenbart eine Vorrichtung zur dreidimensionalen Vermessung von Oberflächen in Hohlräumen. Die Vorrichtung weist ein Endoskop auf, in dessen Schaft ein optischer Abbildungskanal und ein optischer Projektionskanal angeordnet sind, wobei beide Kanäle distal seitlich aus dem Endoskop herausgeführt sind, wobei die optischen Achsen um einen Achswinkel zueinander geneigt sind, und die Austrittsöffnungen der Kanäle voneinander beabstandet sind. Über den Projektionskanal wird eine Sequenz von vorzugsweise sinusförmigen Gitterstrukturen auf die zu vermessende Oberfläche projiziert, und über den Abbildungskanal wird die strukturiert beleuchtete zu vermessende Oberfläche mittels einer Kamera erfasst.

Alle zuvor beschriebenen bekannten Verfahren und Vorrichtungen haben jedoch den Nachteil, dass eine Navigation des Endoskops lediglich anhand eines zuvor durch ein extrakorporales Verfahren bestimmtes Modell realisiert wird, was unter Umständen jedoch keine exakte Korrelation zwischen dem jeweiligen Endo- skopiebild eines Oberflächenareals des Körperinnenraums mit dem zugehörigen Areals des Modells gewährleistet, so dass der Nachteil fortbesteht, dass bei einer endoskopischen Untersuchung die Gefahr besteht, dass bestimmte Bereiche des Körperinnenraums unberücksichtigt bleiben können. Der Erfindung liegt daher die Aufgabe zugrunde, ein Verfahren und eine Vorrichtung der eingangs genannten Art bereitzustellen, die zuverlässiger eine vollständige Bilderfassung der gesamten Oberfläche des Körperinnenraums ermöglichen.

Erfindungsgemäß wird diese Aufgabe durch ein Verfahren zum Erstellen zumindest eines Ausschnitts eines virtuellen 3D-Modells eines Körperinnenraums, insbesondere eines Hohlorgans, aus Daten gelöst, die durch zumindest ein in den Körperinnenraum eingeführtes Endoskop bereitgestellt werden, wobei die Daten umfassen:
- zumindest eine Position und Orientierung des Endoskops, wobei der zumindest einen Position und Orientierung
- zumindest ein Abstand zwischen dem Endoskop und zumindest einem Punkt auf einer Oberfläche des Körperinnenraums, wobei dieser Abstand mittels eines am Endoskop angeordneten oder in dieses integriertes Abstandsmesssystem erfasst wird, und
- zumindest ein Endoskopiebild der Oberfläche des Körperinnenraums im Bereich des zumindest einen Punktes auf der Oberfläche des Körperinnenraums
zugeordnet sind, wobei aus den genannten Daten zu mehreren unterschiedlichen Positionen und Orientierungen des Endoskops der oder die Ausschnitte des 3D-Modells in Überlagerung mit dem zugehörigen Endoskopiebild erstellt wird bzw. werden.

Hinsichtlich der eingangs genannten Vorrichtung wird die Aufgabe durch eine Vorrichtung zum Erstellen zumindest eines Ausschnitts eines 3D-Modells eines Körperinnenraums, insbesondere eines Hohlorgans, gelöst, mit zumindest einem Endoskop zur Erfassung zumindest eines Endoskopiebildes zumindest eines Ausschnitts einer Oberfläche des Körperinnenraums, einem Lageerfassungssystem zur Erfassung der Position und Orientierung des Endoskops, einem Abstandsmesssystem zur Erfassung zumindest eines Abstands des Endoskops zu zumindest einem Punkt der Oberfläche des Körperinnenraums in Abhängigkeit der Position und Orientierung des Endoskops, wobei das Abstandsmesssystem am Endoskop angeordnet oder in dieses integriert ist, und mit einem Datenverarbeitungssystem, das so ausgebildet ist, dass es aus einer Mehrzahl von aus unterschiedlichen Positionen und Orientierungen des Endoskops durch das Abstandsmesssystem erfassten Punkten auf der Oberfläche des Körperinnenraums den oder die Ausschnitte des virtuellen Modells der Oberfläche des Körperinnenraums erstellt und das zumindest eine Endoskopiebild auf der so erstellte Modell projiziert.

Erfindungsgemäß wird demnach mit zumindest einem Endoskop zumindest ein Endoskopiebild zumindest eines Oberflächenareals des Körperinnenraums erfasst, wobei währenddessen mittels eines Lageerfassungssystems die Position und Orientierung des Endoskops erfasst wird. Ferner wird das zumindest eine Endoskopiebild in einem Datenverarbeitungssystem, das eine Bildverarbeitung beinhaltet, verarbeitet, wobei das zumindest eine Endoskopiebild des zumindest einen Oberflächenareals in dem Datenverarbeitungssystem auf ein entsprechendes Oberflächenareal des virtuellen Modells des Körperinnenraums projiziert wird. Das virtuelle 3D-Modell des Körperinnenraums wird dadurch gewonnen, dass eine Mehrzahl von Endoskopiebildern aus unterschiedlichen Positionen des Endoskops und zu jedem Endoskopiebild zumindest ein Punkt auf der Oberfläche des Körperinnenraums räumlich durch eine Abstandsmessung zwischen dem Endoskop und der Oberfläche des Körperinnenraums, vorzugsweise im Bereich des Bildfeldkegels des Endoskops, erfasst werden, und dass aus den räumlich erfassten Punkten das Modell der Oberfläche des Körperinnenraums erzeugt wird, und wobei das zumindest eine Endoskopiebild anhand der erfassten Position und Orientierung des Endoskops auf das erstellte virtuelle 3D-Modell projiziert wird.

Bei dem erfindungsgemäßen Verfahren und der erfindungsgemäßen Vorrichtung wird demnach das virtuelle 3D-Modell, das heißt die Rekonstruktion der Oberfläche des Körperinnenraums als Computermodell gleichzeitig mit der Erfassung einer Mehrzahl von Endoskopiebildern aus unterschiedlichen Positionen des Endoskops heraus erstellt. Dazu werden mit dem Endoskop nicht nur Endoskopiebilder erfasst, sondern gleichzeitig wird die Position und Orientierung des Endoskops, beispielsweise bezüglich eines festen Referenzpunktes, der beispielsweise durch die Einführöffnung des Endoskops in den Körperinnenraum definiert sein kann, erfasst und außerdem werden die jeweiligen Abstände des Endoskops zu einer Mehrzahl von Punkten auf der Oberfläche des Körperinnenraums erfasst.

Aus der Kenntnis der spezifischen Endoskopdaten, nämlich Blickrichtung bezüglich der Längsachse des Endoskopschaftes und numerischer Apertur des Endoskopobjektivs des Endoskops und den Daten aus der Lageerfassung und Abstandsmessung wird ein virtuelles Modell der Oberfläche des Körperinnenraums im Datenverarbeitungssystem erzeugt, dem die bei den Positions-, Orientierungs- und Abstandsmessungen aufgenommenen Endoskopiebilder überlagert werden. Dadurch entsteht das virtuelle Modell im Datenverarbeitungssystem gleichzeitig mit den Texturierungen der Oberfläche des Gewebes des Körperinnenraums. Mit anderen Worten erfolgen Endoskopiebilderfassung und Ausmessung zur Erstellung des virtuellen Modells des Körperinnenraums in einem Untersuchungsvorgang. Bereits bei der ersten endoskopischen Inspektion des Körperinnenraums werden eine Vielzahl von Koordinaten erhalten, aus der die komplette Geometrie des Körperinnenraums erhalten werden kann, wenn zwischen den gemessenen Koordinaten interpoliert oder extrapoliert wird. Im Fall der Untersuchung der Harnblase können beispielsweise vom Arzt direkt Referenzpunkte, beispielweise die Orifizien, angefahren werden und diese als Stützpunkte für die Interpolation genutzt werden. Die Harnröhrenmündung in die Harnblase kann ebenfalls als weiterer Stützpunkt genutzt werden, und der entsprechende Harnblasenbereich kann daraus extrapoliert werden.

Mit dem erfindungsgemäßen Verfahren und der erfindungsgemäßen Vorrichtung kann das zumindest eine Endoskopiebild in Echtzeit mit dem erstellten virtuellen Modell des Körperinnenraums verknüpft werden.

Zur Bilderfassung der Oberfläche des Körperinnenraums können mehrere Endoskope nacheinander verwendet werden, die sich hinsichtlich ihrer Blickrichtungen in Bezug auf ihre Längsachse unterscheiden; so können beispielsweise Endoskope mit Endoskopoptiken mit 0°, 30°, 60°, 90° und 120°-Optik verwendet werden, oder es kann ein flexibles Endoskop verwendet werden, dessen Blickrichtung sich durch Auslenkung des distalen Endes aus der Längsachse variieren lässt.

Das erfindungsgemäße Verfahren und die erfindungsgemäße Vorrichtung haben des Weiteren den Vorteil, dass sie eine Bilderfassung und -dokumentation ermöglichen, die gegenüber Verschiebungen und Verformungen des Körperinnenraums weitestgehend unabhängig ist, da bei jeder Untersuchung das virtuelle Modell des Körperinnenraums neu aufgenommen werden kann. Im Falle der Untersuchung einer Harnblase hängt nämlich die Lage und Form der Harnblase unter anderem vom Füllstand der Blase und von der Lage des Patienten ab. Durch die Abstandsmessung zwischen Endoskop und zumindest einem Punkt auf der Oberfläche des Körperinnenraums, die permanent durchgeführt wird, können solche Vierschiebungen und Verformungen des Körperinnenraums herausgerechnet werden, so dass bei jeder Untersuchung ein aussagekräftiger Vergleich mit dem Zustand des Körperinnenraums bei einer früheren Untersuchung ermöglicht wird.

In einer bevorzugten Ausgestaltung des Verfahrens werden auf dem virtuellen Modell des Körperinnenraums diejenigen Oberflächenareale der Oberfläche des Körperinnenraums visuell markiert, von denen bereits ein Endoskopiebild erfasst wurde.

Entsprechend ist das Datenverarbeitungssystem der erfindungsgemäßen Vorrichtung so ausgebildet, dass es auf dem virtuellen Modell des Körperinnenraums diejenigen Oberflächenareale visuell markiert, von denen bereits ein Endoskopiebild erfasst wurde.

Durch die Markierung bereits eingesehener Oberflächenareale des Körperinnenraums auf dem Monitor wird dem untersuchenden Arzt die Navigation des Endoskops noch weiter erleichtert, weil er durch die Markierungen unmittelbar erkennen kann, welche Oberflächenareale noch nicht durch ein Endoskopiebild erfasst wurden, die er dann gezielt anfahren kann. Außerdem können die Markierungen in vorteilhafter Weise als Wiederauffindhilfe bestimmter Oberflächenareale des Körperinnenraums, beispielsweise maligner Areale, in späteren Untersuchungen verwendet werden, wodurch eine besonders gezielte reproduzierbare Untersuchung bestimmter Oberflächenareale des Körperinnenraums ermöglicht wird.

Vorzugsweise erfolgt die Markierung der Oberflächenareale des Körperinnenraums, zu denen bereits ein Endoskopiebild erfasst wurde, anhand der Erfassung der Verweildauer der Betrachtung dieser Areale, anhand einer unterschiedlichen Lichtintensität und/oder eines Signals, das bei der Betrachtung eines bestimmten Areals erzeugt wird.

In einer weiteren bevorzugten Ausgestaltung des verfahrens erfolgt die Abstandsmessung zur räumlichen Erfassung der Punkte auf der Oberfläche des Körperinnenraums mittels eines berührungslosen Abstandsmesssystems, das in bevorzugten Ausgestaltungen auf der Basis zumindest eines vom Endoskop emittierten Laserstrahls auf der Basis einer Triangulation oder durch Laufzeitmessung des Laserstrahls, auf der Basis eines vom Endoskop auf die Oberfläche des Körperinnenraums projizierten Musters, beispielsweise eines Streifen-, Gitter- oder Punktmusters, oder auf der Basis vom Endoskop emittierten Ultraschalls durch Laufzeitmessung des Ultraschalls arbeitet.

Es kann insbesondere vorzugsweise vorgesehen sein, dass zu einer Position und Orientierung auch der Abstand zu mehreren Punkten auf der Oberfläche des Körperinnenraums gemessen wird, die dann alle oder teilweise im Blickfeld des Endoskops liegen. Weiterhin ist es bevorzugt, wenn die Abstandsmessungen und/oder Lageerfassungsmessungen in Zeitabständen durchgeführt werden, die kürzer als eine Sekunde sind und vorzugsweise im Bereich von Millisekunden oder darunter liegen, wodurch eine besonders exakte Reproduktion des Körperinnenraums durch das virtuelle Modell erreicht wird. Die Abstandsmessung wird dabei vorzugsweise vom Datenverarbeitungssystem oder einer entsprechenden Steuerung automatisch ausgelöst.

Das Abstandsmesssystem der erfindungsgemäßen Vorrichtung ist vorzugsweise am Endoskop selbst angeordnet, was den Vorteil hat, dass das Abstandsmesssystem stets fest mit einem Punkt bzw. einer Stelle des Endoskops korreliert ist.

weiterhin ist es bevorzugt, wenn das Datenverarbeitungssystem so ausgebildet ist, dass es ein weiteres virtuelles 3D-Modell des Körperinnenraums aus einem durch ein bildgebendes Verfahren mit Tiefenwirkung, beispielsweise Computertomografie, Magnetresonanz, Ultraschall, gewonnen Bild des Körperinnenraums ableitet.

Dabei ist es weiterhin bevorzugt, wenn das virtuelle Modell des Körperinnenraums in dem Datenverarbeitungssystem mit einem durch ein bildgebendes Verfahren mit Tiefenwirkung, beispielsweise Computertomografie, Magnetresonanz oder Ultraschall, gewonnenen Bild des Körperinnenraums lagerichtig überlagert wird.

Hinsichtlich der erfindungsgemäßen Vorrichtung wird dies vorzugsweise dadurch realisiert, dass das Datenverarbeitungssystem so ausgebildet ist, dass es das virtuelle Modell des Körperinnenraums mit einem beispielsweise im Datenverarbeitungssystem hinterlegten durch ein bildgebendes Verfahren mit Tiefenwirkung, beispielsweise Computertomografie, Magnetresonanz, Ultraschall, gewonnenen Bild des Körperinnenraums überlagert.

Hierbei ist von Vorteil, dass gleichzeitig zur optisch erfassten Oberfläche des Körperinnenraums Informationen über die Tiefenausdehnung eines Tumors, der oberflächlich optisch erfasst wurde, über die zugehörigen Daten aus den zuvor genanten bildgebenden Verfahren erhalten werden können.

Zur insbesondere bevorzugten Realisierung einer sogenannten "Look-Ahead-Funktion" ist es weiterhin bevorzugt, wenn das Datenverarbeitungssystem so ausgebildet ist, dass es einen Betrachtungsmodus ermoglicht, mit dem in Blickrichtung des Endoskops vom Endoskopiebild der Oberfläche des Körperinnenraums ausgehend in die lagerichtige 3D-Information aus dem bildgebenden 3D-Verfahren mit Tiefenwirkung weiterbetrachtet werden kann. Dem untersuchenden Arzt wird somit die Möglichkeit an die Hand gegeben, mit einem "virtuellen Endoskop" ausgehend vom endoskopischen Oberflächenbild in das Gewebe hinein zu schauen und dort ein malignes Gewebe auch in der Tiefenausdehnung zu untersuchen und zu bewerten.

Bei einer weiteren bevorzugten Ausgestaltung des Verfahrens wird das Modell der Oberfläche des Körperinnenraums auch aus Endoskopiebildern abgeleitet, die zumindest teilweise unter Fluoreszenzanregung gewonnen wurden.

Mit dieser Maßnahme wird der Vorteil einer verbesserten Tumorvisualisierung bei der Untersuchung des Körperinnenraums erreicht. Mittels einer zuvor applizierten Substanz, die sich in Tumorgewebe stärker anreichert als in gesundem Gewebe, und die optisch zur Fluoreszenz angeregt werden kann, lassen sich somit bei der dreidimensionalen Darstellung der Oberfläche des Körperinnenraums Tumore hervorgehoben visualisieren.

Dabei kann es vorgesehen sein, dass nur die Bereiche der Oberfläche des Körperinnenraums unter Fluoreszenzanregung im virtuellen Modell eingeblendet werden, die gewebespezifisch abnorm sind.

In einer weiteren bevorzugten Ausgestaltung des Verfahrens wird das zumindest eine Endoskopiebild in Überlagerung mit dem virtuellen Modell der Oberfläche des Körperinnenraums wahlweise dreidimensional oder zweidimensional dargestellt, wobei vorzugsweise die zweidimensionale Darstellung aus der dreidimensionalen Darstellung durch ein rechnergestütztes Projektionsverfahren hergeleitet wird. Ein solches Verfahren ist beispielsweise die Mercator-Projektion.

Die zweidimensionale Darstellung hat zusätzlich zu der dreidimensionalen Darstellung den Vorteil einer verbesserten Übersichtlichkeit der Darstellung, da die gesamte Oberfläche des Körperinnenraums dann in einem Bild in einer Ebene dargestellt ist.

In einer weiteren bevorzugten Ausgestaltung der erfindungsgemäßen Vorrichtung weist das Lageerfassungssystem zur Erfassung der Position und Orientierung des Endoskops ein endoskopfestes Trägheitssensorsystem auf.

Mit dem Trägheitssensorsystem werden ausgehend von einem Referenzpunkt, an dem sich das Endoskop zu Beginn des Verfahrens befindet, alle Bewegungen, das heißt alle Positionsänderungen, sowie alle Lageänderungen des Endoskops durch Beschleunigungsmessung erfasst und daraus die aktuelle Position und Orientierung des Endoskops ermittelt. Die Verwendung eines Trägheitssensorsystems zur Lageerfassung des Endoskops hat den Vorteil, dass ein solches System autark ist, das heißt ohne extrakorporale Teilsysteme auskommt, wie es beispielsweise bei einem Lageerfassungssystem mit elektromagnetischen Spulen, deren Lage und Orientierung in einem externen Magnetfeld ermittelt wird, der Fall ist.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausgewählte Ausführungsbeispiele werden hiernach mit Bezug auf die beigefügten Zeichnungen beschrieben. Es zeigen:
- Figur 1: eine äußerst schematische Darstellung einer Vorrichtung zum Erstellen zumindest eines Abschnitts eines virtuellen 3D-Modells eines Körperinnenraums;
- Figur 2: die Vorrichtung in Figur 1 unter Weglassung von Teilen in einem gegenüber Figur 1 veränderten Arbeitszustand;
- Figur 3: ein Blockschaltdiagramm des Verfahrens zum Erstellen zumindest eines Ausschnitts eines virtuellen 3D-Modells des Körperinnenraums; und
- Figur 4: eine in eine Ebene projizierte Darstellung, die aus einem dreidimensionalen Modell der Oberfläche des Körperinnenraums gewonnen wurde.

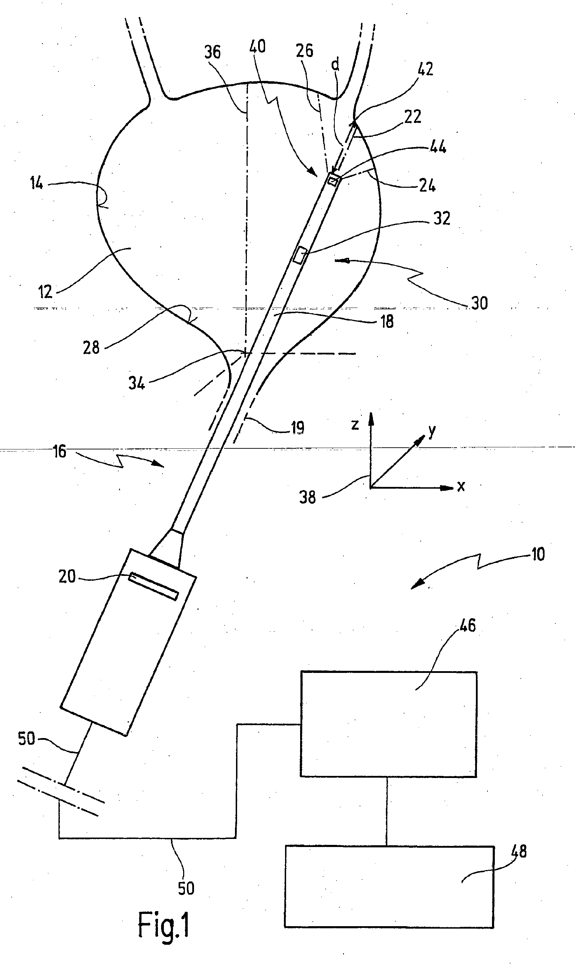

In Figur 1 ist eine mit dem allgemeinen Bezugszeichen 10 versehene Vorrichtung zur zumindest ausschnittsweisen Erstellung eines 3D-Modells eines Körperinnenraums 12 dargestellt. In dem gezeigten Ausführungsbeispiel ist der Körperinnenraum 12 die Harnblase eines Patienten. Mit der Vorrichtung 10 wird genauer gesagt eine Oberfläche 14 des Körperinnenraums 12 endoskopisch räumlich erfasst. Die Vorrichtung 10 ermöglicht eine endoskopische Dokumentation der vorzugsweise gesamten Oberfläche 14 des Körperinnenraums 12, wie hiernach noch beschrieben wird.

Die Vorrichtung 10 weist ein Endoskop 16 mit einem Schaft 18 auf, das bzw. der in den Körperinnenraum 12 einführbar ist. Im vorliegenden Ausführungsbeispiel, in dem der Körperinnenraum 12 der Innenraum einer Harnblase ist, wird der Schaft 18 über den Harnröhre 19 in die Harnblase eingeführt.

Das Endoskop 16 kann ein sogenanntes starres Endoskop sein, das heißt in diesem Fall ist der Schaft 18 starr, oder das Endoskop 16 kann ein flexibles Endoskop sein, wobei dann der Schaft 18 flexibel ausgestaltet ist. Im gezeigten Ausführungsbeispiel ist das Endoskop 16 ein starres Endoskop.

Vorzugsweise ist das Endoskop 16 ein Video-Endoskop, das mit einer Kamera 20 ausgerüstet ist, die, wie im vorliegenden Ausführungsbeispiel dargestellt, in einem proximalen Bereich des Endoskops 16 angeordnet ist, aber auch im distalen Bereich des Endoskop angeordnet sein kann. Im Falle der Anordnung der Kamera 20 im proximalen Bereich weist das Endoskop 16 im Schaft 18 ein Bildübertragungssystem, beispielsweise in Form einer Relaislinsenanordnung oder eines geordneten Lichtfaserbündels, auf.

Das Endoskop 16 ist durch seine Blickrichtung 22 und durch sein Bild- oder Blickfeld charakterisiert, das wiederum durch die numerische Apertur des Objektivs des Endoskops 16 bestimmt ist. In Figur 1 ist der Öffnungswinkel des Blickfeldes durch Begrenzungslinien 24 und 26 veranschaulicht. Das von dem Endoskop 16 erfasste Endoskopbild der Oberfläche 14 des Körperinnenraums 12 ist im Wesentlichen kreisförmig bzw. in Abhängigkeit der Form der Oberfläche 14 kegelschnittförmig.

Die Vorrichtung 10 kann weitere Endoskope (nicht dargestellt) aufweisen, die wechselweise im Austausch mit dem Endoskop 16 in den Körperinnenraum 12 eingeführt werden können, wobei sich diese Endoskope dann durch ihre Blickrichtung 22 unterscheiden. Beispielsweise kann an Stelle des Endoskops 16, dessen Blickrichtung 22 eine 0°-Blickrichtung ist, durch ein Endoskop mit einer 120°-Optik ausgetauscht werden, um beispielsweise auch ein rückwärtig gelegenes Oberflächenareal 28 durch ein Endoskopiebild erfassen zu können. Dies kann jedoch auch dadurch erreicht werden, dass die Vorrichtung 10 an Stelle eines oder mehrerer starrer Endoskope ein flexibles Endoskop aufweist, dessen Blickrichtung durch Auslenken des distalen Endes des Schaftes variiert werden kann.

Die Vorrichtung 10 weist weiterhin ein Lageerfassungssystem 30 zur Erfassung der Position und Orientierung des Endoskops 16 auf. Das Lageerfassungssystem 30 weist ein Trägheitssensorsystem 32 auf, das lagefest am Endoskop 16, beispielsweise wie in Figur 1 dargestellt ist, in einem distalen Bereich des Schafts 18 des Endoskops 16 angeordnet ist. Das Trägheitssensorsystem 32 kann außenseitig am Schaft 18 oder im Innern des Schafts 18 angeordnet sein.

Das Trägheitssensorsystem 32 erfasst die Postition und Orientierung des Endoskops 16, indem es Sensoren aufweist, die Drehungen und Translationen im Raum detektieren, wobei das Trägheitssensorsystem 32 in der Lage ist, alle sechs translatorischen und rotatorischen Freiheitsgrade des Endoskops 16 zu detektieren. Als Referenzpunkt 34 für die Erfassung der Positionen des Endoskops 16 kann beispielsweise im vorliegenden Fall die Mündung der Harnröhre 19 in die Harnblase verwendet werden. Als Referenzachse 36 für die Erfassung der Orientierung des Endoskops 16 kann beispielsweise die Mittelachse des Körperinnenraums 12, hier der Harnblase, verwendet werden, und für die Drehlage des Endoskops um seine Längsachse eine schaftfeste Achse.

Ein entsprechendes Referenzkoordinatensystem 38 ist in Figur 1 separat dargestellt.

Die Vorrichtung 10 weist weiterhin ein Abstandsmesssystem 40 zur Erfassung zumindest eines jeweiligen von der Position und Orientierung des Endoskops 16 abhängigen Abstandes des Endoskops 16 zu einem Punkt 42 auf der Oberfläche 14 des Körperinnenraums 12 auf. Das Abstandsmesssystem 40 ist ein berührungsloses am Endoskop 16 angeordnetes Abstandsmesssystem, das beispielsweise eine Laserlichtquelle 44 aufweist, die vorzugsweise in Richtung der Blickrichtung 22 zumindest einen Laserstrahl auf die Oberfläche 14 des Körperinnenraums 12 richtet, das heißt die Abstandsmessung erfolgt vorzugsweise in der Mitte des durch die Linien 24 und 26 begrenzten Bildkegels des Endoskops 16. Mit dem zuvor erwähnten Laserlichtstrahl wird ein Abstand d beispielsweise auf der Basis einer Triangulation oder durch Laufzeitmessung des Laserlichtstrahls von der Laserlichtquelle 44 zum Punkt 42 und wieder zurück zur Laserlichtquelle, wo ein entsprechender Sensor angebracht ist, erfasst.

Andere bevorzugte Möglichkeiten der Ausgestaltung des Abstandsmesssystems 40 können darin bestehen, dass mit der Laserlichtquelle 44 ein Muster auf die Oberfläche 14 des Körperinnenraums 12 projiziert wird, beispielsweise ein Streifen-, Gitter- oder Punktmuster. An Stelle einer Laserlichtquelle 44 kann jedoch auch am Endoskop 16 eine Ultraschallquelle angeordnet sein, wobei dann das Abstandsmesssystem 40 den Abstand d mittels des von der Ultraschallquelle emittierten Ultraschalls durch Laufzeitmessung des Ultraschalls ähnlich zur Laufzeitmessung mittels eines Laserstrahls durchführt.

Das Abstandsmesssystem nach einer der zuvor genannten Arten kann insbesondere so modifiziert werden, dass es gleich zu mehreren Punkten innerhalb des Bildfeldkegels den Abstand des Endoskops 16 zur Oberfläche 14 erfassen kann.

Die Vorrichtung 10 weist weiterhin ein Datenverarbeitungssystem 46 sowie einen Monitor 48 auf.

In dem Datenverarbeitungssystem 46 laufen die Signale bzw. Daten des Lageerfassungssystems 30, des Abstandsmesssystems 40 und der Kamera 20, die ein Endoskopiebild in der zugehörigen Position und Orientierung des Endoskops 16 und in dem zugehörigen Abstand d empfängt, über beispielsweise eine Signalleitung 50 zusammen.

Das Datenverarbeitungssystem 46 ist dazu so ausgebildet, dass es aus einer Mehrzahl von aus unterschiedlichen Positionen und Orientierungen des Endoskops 16 durch das Abstandsmesssystem 40 räumlich erfassten Punkten der Oberfläche 14 des Körperinnenraums 12 zumindest ausschnittsweise ein virtuelles Modell der Oberfläche 14 erzeugt und dabei die optisch erfassten Endoskopiebilder auf das so erzeugte Modell projiziert, so dass dann auf dem Monitor 48 die dreidimensional erfasste Oberfläche 14 des Körperinnenraums 12 mit endoskopischer Bildinformation aufgezeichnet wird. Die vorzugsweise gesamte endoskopisch dreidimensional erfasste Oberfläche 14 kann dann zur Dokumentation in dem Datenverarbeitungssystem 46 abgespeichert werden, um diese abgespeicherte Information für spätere Untersuchungen des Körperinnenraums 12 wieder zu verwenden, um beispielsweise einen Krankheitsverlauf oder einen Therapieverlauf hinsichtlich seiner Entwicklung beurteilen zu können.

Bei dem Verfahren zur zumindest ausschnittsweisen 3D-Modellerstellung des Körperinnenraums 12, genauer gesagt dessen Oberfläche 14, wird also entsprechend das virtuelle 3D-Modell der Oberfläche 14 des Körperinnenraums 12 dadurch gewonnen, dass eine Mehrzahl von Endoskopiebildern aus unterschiedlichen Positionen des Endoskops 16 erfasst werden, wobei zu jedem Endoskopiebild auch mittels des Lageerfassungssystems 30 die Position und Orientierung des Endoskops 16 erfasst wird.

Des Weiteren wird zu jedem Endoskopiebild und erfasster Position und Orientierung des Endoskops 16 mittels des Abstandsmesssystems 40 der Abstand d zu dem Punkt 42 auf der Oberfläche 14 des Körperinnenraums 12 erfasst.

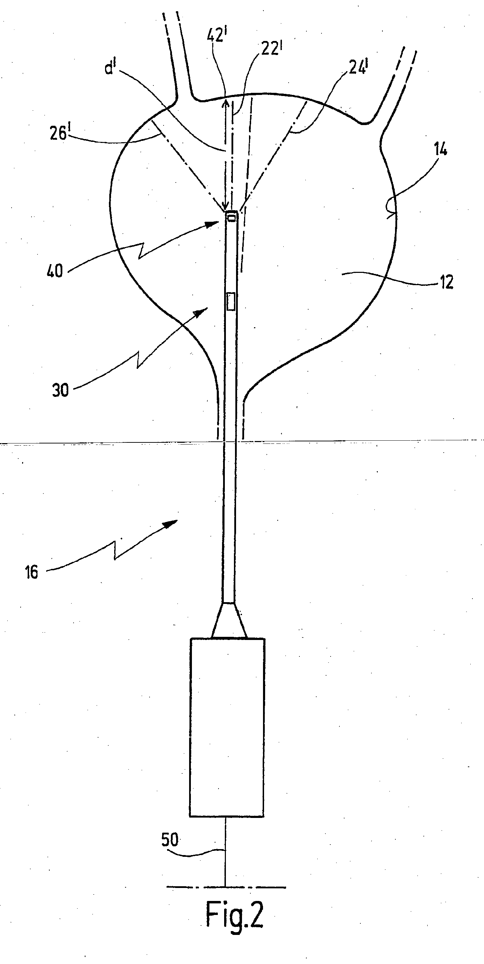

Zur Veranschaulichung ist das Endoskop 16 in Figur 2 in einer gegenüber Figur 1 veränderten Position und Orientierung im Körperinnenraum 12 dargestellt, wobei diese Orientierung und Position des Endoskops 16 wiederum mittels des Lageerfassungssystems 30 erfasst wird, und wobei gleichzeitig mittels des Abstandsmesssystems 40, wieder in Richtung der Blickrichtung 22 ein Abstand d' zu einem Punkt 42' auf der Oberfläche 14 des Körperinnenraums 12 erfasst wird. Ebenso wird das zugehörige Endoskopiebild des innerhalb der Begrenzung 24, 26 des Blickfeldes liegenden Oberflächenareals erfasst.

Aus den zuvor genannten Daten, nämlich den verschiedenen Positionen und Orientierungen des Endoskops und den zugehörigen Endoskopiebildern und entsprechenden Abständen d des Endoskops 16 zur Oberfläche 14 des Körperinnenraums 12 wird dann das dreidimensionale virtuelle Modell der Oberfläche 14 des Körperinnenraumes 12 erstellt, und das jeweilig erfasste Endoskopiebild wird dann anhand der erfassten Position und Orientierung des Endoskops 16, die mit dem Lageerfassungssystem 30 erfasst wird, auf das entsprechende Oberflächenareal des virtuellen Modells des Körperinnenraums 12 projiziert. Sich gegebenenfalls überlappende Endoskopiebilder werden im Datenverarbeitungssystem 46 beispielsweise lagegerecht übereinander gelegt oder so zugeschnitten, dass kein Überlapp mehr besteht.

Die Abstandsmessung mittels des Abstandsmesssystems 40 wird dabei möglichst automatisch ausgelöst, beispielsweise in Zeitabständen von wenigen Millisekunden. Auch die Lageerfassung mittels des Lageerfassungssystems 30 wird vorzugsweise synchronisiert mit der Abstandsmessung automatisch ausgelöst.

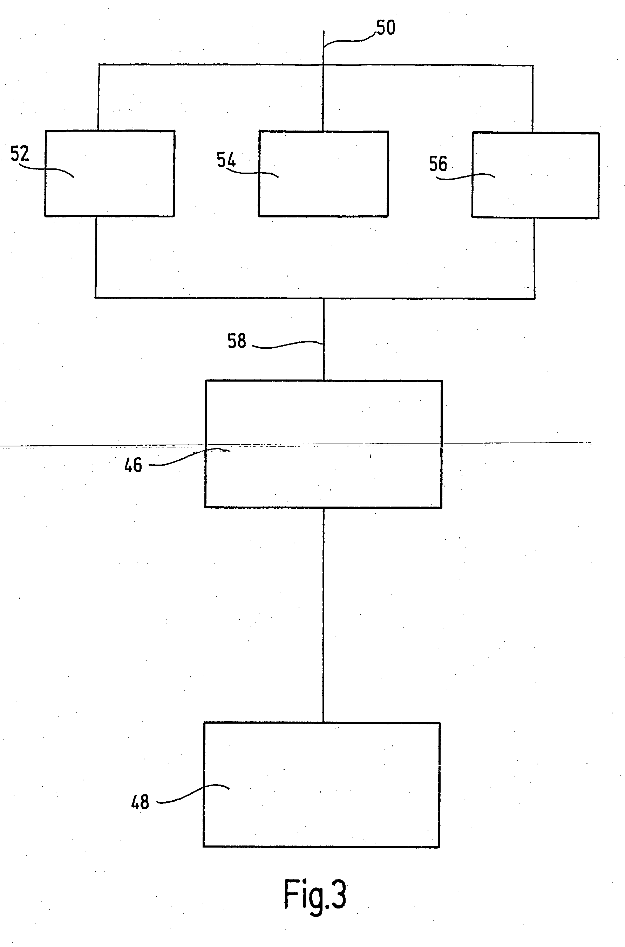

In Figur 3 ist das Verfahren in einem Blockschaltbild grob skizziert, wobei mit 52 die einzelne Lageerfassung des Endoskops 16, mit 54 die einzelne Abstandsmessung des Abstandes d des Endoskops 16 zur Oberfläche 14 des Körperinnenraums 12 und mit 56 das jeweils während der Lageerfassung 52 und Abstandsmessung 54 erfasste Endoskopiebild 56 bezeichnet ist. Diese Informationen werden dann über eine Signalleitung 58 dem Datenverarbeitungssystem 46 zugespeist, in der diese Informationen so verarbeitet werden, dass das bereits erwähnte virtuelle dreidimensionale Modell der Oberfläche 14 des Körperinnenraums 12 erstellt und das Endoskopiebild 56, das von einem Oberflächenareal der Oberfläche 14 stammt, auf das entsprechende Oberflächenareal bzw. den entsprechenden Abschnitt des Modells der Oberfläche 14 projiziert wird.

Auf dem Monitor 48 wird dann die dreidimensional rekonstruierte Oberfläche 14 des Körperinnenraums 12 zusammen mit der darauf projizierten endoskopischen Bildinformation dargestellt.

Des Weiteren ist vorzugsweise vorgesehen, dass auf dem so erzeugten virtuellen Modell der Oberfläche 14 des Körperinnenraums 12 diejenigen Oberflächenareale bzw. Ausschnitte visuell markiert werden, von denen bereits ein Endoskopiebild erfasst wurde. Auf diese Weise kann der Arzt feststellen, welche Oberflächenareale der Oberfläche 14 er bereits eingesehen hat, und welche nicht.

Die Markierung, beispielsweise farbliche oder Hell-Dunkel-Markierung, der Oberflächenareale 14 des Körperinnenraums 12, zu denen bereits ein Endoskopiebild erfasst wurde, kann anhand der Erfassung der Verweildauer der Betrachtung dieser Oberflächenareale, anhand einer unterschiedlichen Lichtintensität und/oder anhand eines Signals, das bei der Betrachtung eines bestimmten Oberflächenareals erzeugt wird, erfolgen. Die Markierung der Oberflächenareale anhand der Verweildauer der Betrachtung erhöht die Sicherheit bei der Untersuchung des Körperinnenraums 12, weil über die Verweildauer beurteilt werden kann, ob das entsprechende Oberflächenareal vom Arzt tatsächlich eingehend inspiziert wurde, oder ob das Oberflächenareal vom Endoskop 16 nur kursorisch oder gar nur zufällig überstrichen wurde.

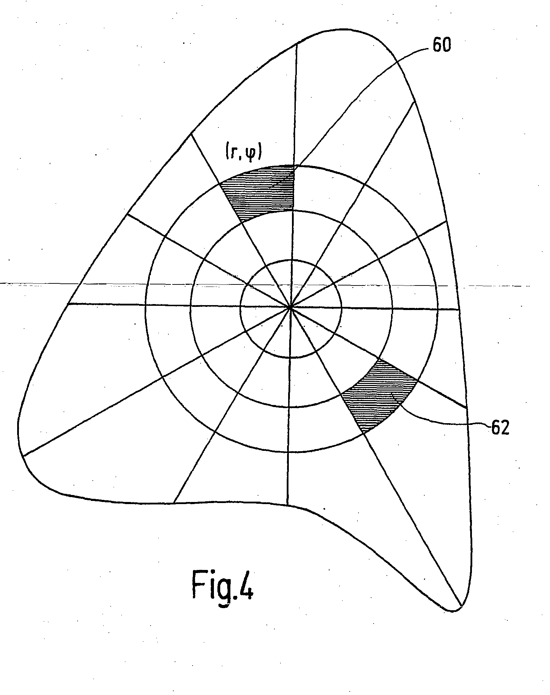

In Figur 4 ist als Beispiel das Monitorbild des Monitors 48 dargestellt. Wie bereits erwähnt, kann die Darstellung der dreidimensional rekonstruierten Oberfläche 14 des Körperinnenraums 12 auf dem Monitor 48 als dreidimensionale Darstellung, das heißt perspektivisch, erfolgen, oder es kann, wie in Figur 4 dargestellt ist, die dreidimensional rekonstruierte Oberfläche 14 des Körperinnenraums 12 in eine Ebene projiziert (beispielsweise Mercator-Projektion) und diese dann zweidimensional auf dem Bildschirm dargestellt werden.

In Figur 4 sind auch beispielhaft zwei markierte Oberflächenareale 60 und 62 dargestellt, für die bereits ein Endoskopiebild erfasst wurde.

Des Weiteren kann es vorzugsweise vorgesehen werden, dass das virtuelle Modell samt darauf projizierten Endoskopbildern der Oberfläche 14 des Körperinnenraums 12 in dem Datenverarbeitungssystem 46 zusätzlich mit einem durch ein bildgebendes Verfahren mit Tiefenwirkung, beispielsweise Computertomografie, Magnetresonanz, Ultraschall, gewonnenen Bild des Körperinnenraums 12 überlagert wird, um so zusätzlich zu der optischen Oberflächeninformation über den Körperinnenraum 12 Tiefeninformationen, beispielsweise Aufschluss über die Tiefe eines malignen Bereichs des Gewebes unterhalb der Oberfläche 14 des Körperinnenraums 12 oder zu benachbartem Gewebe und Organen zu erhalten. Dabei kann das Datenverarbeitungssystem 46 so ausgelegt sein, dass es aus den Informationen, die durch ein bildgebendes Verfahren mit Tiefenwirkung erhalten werden, ein weiteres virtuelles 3D-Modell des Körperinnenraums 12 erstellt. Das Datenverarbeitungssystem 46 ist dann vorzugsweise so ausgebildet, dass auf dem Monitor 48 ein Betrachtungsmodus angeboten wird, bei dem in Blickrichtung des Endoskops 16 ausgehend von dem endoskopischen Oberflächenbild in die lagerichtige 3D-Information, die aus dem bildgebenden Verfahren mit Tiefenwirkung erhalten wird, weiterbetrachtet werden kann. Auf diese Weise wird eine sogenannte "Zook-Ahead-Funktion" realisiert.

Des Weiteren kann ebenfalls vorzugsweise vorgesehen werden, das zuvor erwähnte virtuelle Modell der Oberfläche 14 des Körperinnenraums 12 zumindest teilweise in Fluoreszenzanregung zu gewinnen, wie es bei der sogenannten photodynamischen Diagnose der Fall ist. Zusätzlich zu der dreidimensionalen Rekonstruktion und endoskopischen visuellen Darstellung der Oberfläche 14 des Körperinnenraums 12 können so aufgrund unterschiedlicher Fluoreszenzsignale aus unterschiedlichen Oberflächenarealen der Oberfläche 14 Tumore deutlicher visualisiert werden. Wenn das virtuelle 3D-Modell der Oberfläche 14 des Körperinnenraums 12 aus Endoskopiebildern abgeleitet wird, die zumindest teilweise unter Fluoreszenzanregung gewonnen wurden, kann vorzugsweise vorgesehen sein, dass nur die Bereiche unter Fluoreszenzanregung im virtuellen Modell eingeblendet werden, die gewebespezifisch abnorm, das heißt pathologisch, sind.

## Patentansprüche

1. Verfahren zum Erstellen zumindest eines Ausschnitts eines virtuellen 3D-Modells eines Körperinnenraums (12), insbesondere eines Hohlorgans, aus Daten, die durch zumindest ein in den Körperinnenraum (12) eingeführtes Endoskop (16) bereitgestellt werden, wobei die Daten umfassen:
zumindest eine Position und Orientierung des Endoskops, (16),
**dadurch gekennzeichnet, dass** der zumindest einen Position pnd Orientierung
- zumindest ein Abstand zwischen dem Endoskop (16) und zumindest einem Punkt (42, 42') auf einer Oberfläche (14) des Körperinnenraums (12), wobei dieser Abstand mittels eines am Endoskop (16) angeordneten oder in dieses integriertes Abstandsmesssystem (40) erfasst wird, und
- zumindest ein Endoskopiebild der Oberfläche (14) des Körperinnenraums (12) im Bereich des zumindest einen Punktes auf der Oberfläche (14) des Körperinnenraums (12)
zugeordnet sind,
wobei aus den genannten Daten zu mehreren unterschiedlichen Positionen und Orientierungen des Endoskops (16) der oder die Ausschnitte des 3D-Modells in Überlagerung mit dem zugehörigen Endoskopiebild erstellt wird bzw. werden.

2. Verfahren nach Anspruch 1, wobei an dem virtuellen Modell des Körperinnenraums (12) diejenigen Oberflächenareale (60, 62) der Oberfläche (14) des Körperinnenraums (12) visuell markiert werden, von denen bereits ein Endoskopie-bild erfasst wurde.

3. Verfahren nach Anspruch 2, wobei die Markierung der Oberflächenareale (60, 62) des Körperinnenraums (12), zu denen bereits ein Endoskopiebild erfasst wurde, anhand der Erfassung der Verweildauer der Betrachtung dieser Oberflächenareale, anhand einer unterschiedlichen Lichtintensität und/oder anhand eines Signals, das bei der Betrachtung eines bestimmten Oberflächenareals erzeugt wird, erfolgt.

4. Verfahren nach einem der Ansprüche 1 bis 3, wobei der zumindest eine Abstand zwischen dem Endoskop (16) und dem zumindest einen Punkt (42, 42') auf der Oberfläche (14) des Körperinnenraums (12) mittels zumindest eines vom Eindoskop (16) emittierten Laserstrahls beispielsweise auf der Basis einer Triangulation stereoskopisch oder durch Laufzeitmessung des Laserstrahls erfasst wird.

5. Verfahren nach einem der Ansprüche 1 bis 4, wobei der zumindest eine Abstand zwischen dem Endoskop (16) und dem zumindest einen Punkt (42, 42') auf der Oberfläche (14) des Körperinnenraums (12) mittels eines vom Endoskop (16) auf die Oberfläche (14) des Körperinnenraums (12) projizierten Musters erfasst wird.

6. Verfahren nach einer der Ansprüche 1 bis 5, wobei der zumindest eine Abstand zwischen dem Endoskop (16) und dem zumindest einen Punkt (42, 42') auf der Oberfläche (14) des Körperinnenraums (12) mittels vom Endoskop (16) emittierten Ultraschalls durch Laufzeitmessung des Ultraschalls erfasst wird.

7. Verfahren nach einem der Ansprüche 1 bis 6, wobei die Abstandsmessung in Zeitabständen durchgeführt wird, die kürzer als eine Sekunde sind, vorzugsweise im Bereich einer Millisekunde oder darunter liegen.

8. Verfahren nach einem der Ansprüche 1 bis 7, wobei der zumindest eine Abschnitt des virtuellen Modells des Körperinnenraums (12) mit dem zumindest einen Endoskopiebild mit einem durch ein bildgebendes Verfahren mit Tiefenwirkung, beispielsweise Computertomografie, Magnetresonanz und/oder Ultraschall, gewonnenen Bild des Körperinnenraums (12) überlagert wird.

9. Verfahren nach einem der Ansprüche 1 bis 8, wobei das virtuelle Modell der Oberfläche (14) des Körperinnenraums (12) aus Endoskopiebildern abgeleitet wird, die zunächst teilweise unter Fluoressenzanregung gewonnen werden.

10. Verfahren nach Anspruch 9, wobei nur diejenigen Oberflächenareale unter Fluoreszenzanregung im virtuellen Modell eingeblendet werden, die gewebespezifisch abnorm sind.

11. Verfahren nach einem der Ansprüche 1 bis 10, wobei das virtuelle Modell von einer 3D-Darstellung in eine 2D-Darstellung umgerechnet wird.

12. Vorrichtung zum Erstellen zunächst eines Ausschnitts eines 3D-Modells eines Körperinnenraums (12), insbesondere eines Hohlorgans, entsprechend einem Verfahren nach einem der Ansprüche 1 bis 11, mit zumindest einem Endoskop (16) zur Erfassung zumindest eines Endoskopiebildes zumindest eines Oberflächenareals einer Oberfläche (14) des Körperinnenraums (12), einem Lageerfassungssystem (30) zur Erfassung der Position und Orientierung des Endoskops (16), **gekennzeichnet durch** ein Abstandsmesssystem (40) zur Erfassung zumindest eines Abstands des Endoskops (16) zu zumindest einem Punkt (42, 42') der Oberfläche (14) des Körperinnenraums (12) in Abhängigkeit der Position und Orientierung des Endoskops (16), wobei das Abstandsmesssystem (40) am Endoskop (16) angeordnet oder in dieses integriert ist, und ein Datenverarbeitungssystem (46), das so ausgebildet ist, dass es aus einer Mehrzahl von aus unterschiedlichen Positionen und Orientierungen des Endoskops (16) **durch** das Abstandsmesssystem (40) erfassten Punkten (42, 42') auf der Oberfläche (14) des Körperinnenraums (12) den oder die Ausschnitte des virtuellen Modells der Oberfläche (14) des Körperinnenraums (12) erstellt und das zumindest eine Endoskopiebild auf das so erstellte Modell projiziert.

13. Vorrichtung nach Anspruch 12, **dadurch gekennzeichnet, dass** das Datenverarbeitungssystem (46) so ausgebildet ist, dass es auf dem virtuellen Modell des Körperinnenraums (12) diejenigen Oberflächenareale visuell markiert, von dienen bereits ein Endoskopiebild erfasst wurde.

14. Vorrichtung nach Anspruch 12 oder 13, wobei das Datenverarbeitungssystem (46) so ausgebildet ist, dass es ein weiteres virtuelles 3D-Modell des Körperinnenraums (12) aus einem durch ein bildgebendes Verfahren mit Tiefenwirkung, beispielsweise computertomografie, Magnetresonanz, Ultraschalt, gewonnenen Bild des Körperinnenraums ableitet.

15. Vorrichtung nach Anspruch 14, wobei das Datenverarbeitungssystem (46) so ausgebildet ist, dass das virtuelle Modell, das aus der endoskopischen Bildinformation abgeleitet ist, dem virtuellen Modell, das aus der Information des bildgebenden Verfahrens mit Tiefenwirkung gewonnen ist, lagerichtig überlagert wird.

16. Vorrichtung nach Anspruch 14 oder 15, wobei das Datenverarbeitungssystem so ausgebildet ist, dass ein Betrachtungsmodus ermöglicht wird, in dem in Blickrichtung des Endoskops (16) vom Endoskopiebild der Oberfläche (14) des Körperinnenraums (12) ausgehend in die lagerichtige 3D-Information des bildgebenden Verfahrens mit Tiefenwirkung weiterbetrachtet werden kann.

17. Vorrichtung nach einem der Ansprüche 12 bis 16, wobei das Lageerfassungssystem (30) ein endoskopfestes Trägheitssensorsystem (32) aufweist.

18. Vorrichtung nach einem der Ansprüche 12 bis 17, wobei das Abstandsmesssystem (40) ein berührungsloses Abstandsmesasystem ist.

## Claims

1. A method for generating at least one section of a virtual 3D model of a body interior (12), in particular a hollow organ, from data that are provided by at least one endoscope (16) introduced into the body interior (12), the data comprising:
- at least one position and orientation of the endoscope (16),
**characterized in that** the at least one position and orientation being assigned
- at least one distance between the endoscope (16) and at least one point (42, 42') on a surface (14) of the body interior (12), wherein this distance is acquired by means of a distance measurement system (40) arranged on the endoscope (16) or integrated in same,
- at least one endoscopic image of the surface (14) of the body interior (12) in the region of the at least one point on the surface (14) of the body interior (12),
the said data relating to a number of different positions and orientations of the endoscope (16) being used to generate the section or sections of the 3D model in superposition with the associated endoscopic image.

2. The method of claim 1, wherein those surface areas (60, 62) of the surface (14) of the body interior (12) of which an endoscopic image has already been acquired are visually marked on the virtual model of the body interior (12).

3. The method of claim 2, wherein the marking of the surface areas (60, 62) of the body interior (12) in relation to which an endoscopic image has already been acquired is performed with the aid of the acquisition of the hold time for viewing these surface areas, with the aid of a different light intensity and/or with the aid of a signal that is generated during viewing of a specific surface area.

4. The method of anyone of claims 1 through 3, wherein the at least one distance between the endoscope (16) and the at least one point (42, 42') on the surface (14) of the body interior (12) is acquired by means of at least one laser beam emitted by the endoscope (16), for example stereoscopically on the basis of a triangulation, or by measuring the transit time of the laser beam.

5. The method of anyone of claims 1 through 4, wherein the at least one distance between the endoscope (16) and the at least one point (42, 42') on the surface (14) of the body interior (12) is acquired by means of a pattern projected by the endoscope (16) onto the surface (14) of the body interior (12).

6. The method of anyone of claims 1 through 5, wherein the at least one distance between the endoscope (16) and the at least one point (42, 42') on the surface (14) of the body interior (12) is acquired by means of ultrasound emitted by the endoscope (16), by measuring the transit time of the ultrasound.

7. The method of anyone of claims 1 through 6, wherein the distance measurement is carried out in time intervals that are shorter than one second, preferably in the range of a millisecond or there below.

8. The method of anyone of claims 1 through 7, wherein the at least one section of the virtual model of the body interior (12) is superposed with the at least one endoscopic image with an image of the body interior (12) obtained by an imaging method having a depth effect, for example computed tomography, magnetic resonance and/or ultrasound.

9. The method of anyone of claims 1 through 8, wherein the virtual model of the surface (14) of the body interior (12) is derived from endoscopic images that are firstly obtained partially under fluorescence excitation.

10. The method of claim 9, wherein only those surface areas under fluorescence excitation that are tissue-specific abnormal are inserted in the virtual model.

11. The method of anyone of claims 1 through 10, wherein the virtual model is converted from a 3D display into a 2D display.

12. An apparatus for generating firstly a section of a 3D model of a body interior (12), in particular a hollow organ, according to a method according to any one of claims 1 through 11, comprising at least one endoscope (16) for acquiring at least one endoscopic image of at least one surface area of a surface (14) of the body interior (12), a position acquisition system (30) for acquiring the position and orientation of the endoscope (16), **characterized by** a distance measurement system (40) for acquiring at least one distance of the endoscope (16) in relation to at least one point (42, 42') of the surface (14) of the body interior (12) as a function of the position and orientation of the endoscope (16), wherein the distance measurement system (40) is arranged on the endoscope or integrated in same, and a data processing system (46) that is designed such that it uses a plurality of points (42, 42'), acquired from different positions and orientations of the endoscope (16) by the distance measurement system (40), on the surface (14) of the body interior (12) to generate the section or sections of the virtual model of the surface (14) of the body interior (12), and projects the at least one endoscopic image onto the model thus generated.

13. The apparatus of claim 12, wherein the data processing system (46) is designed such that it marks visually on the virtual model of the body interior (12) those surface areas of which an endoscopic image has already been acquired.

14. The apparatus of claim 12 or 13, wherein the data processing system (46) is designed such that it derives a further virtual 3D model of the body interior (12) from an image of the body interior obtained by an imaging method having a depth effect, for example computed tomography, magnetic resonance, ultrasound.

15. The apparatus of claim 14, wherein the data processing system (46) is designed such that the virtual model that is derived from the endoscopic image information is superposed in the correct position on the virtual model that is obtained from the information of the imaging method having a depth effect.

16. The apparatus of claim 14 or 15, wherein the data processing system is designed so as to enable a viewing mode in which, starting from the endoscopic image of the surface (14) of the body interior (12), it is possible to continue to view in the viewing direction of the endoscope (16) the positionally correct 3D information of the imaging method having a depth effect.

17. The apparatus of anyone of claims 12 through 16, wherein the position acquisition system (30) has an inertial sensor system (32) fixed to the endoscope.

18. The apparatus of anyone of claims 12 through 17, wherein the distance measurement system (40) is a contactless distance measurement system.

## Revendications

1. Procédé de création d'au moins un extrait d'un modèle 3D virtuel d'une cavité du corps (12), en particulier d'un organe creux, à partir de données qui sont mises à disposition par au moins un endoscope (16) introduit dans la cavité du corps (12), dans lequel les données comprennent :
- au moins une position et une orientation de l'endoscope (16),
**caractérisé en ce que**
- au moins un écart entre l'endoscope (16) et au moins un point (42, 42') sur une surface (14) de la cavité du corps (12), dans lequel cet écart est détecté au moyen d'un système de mesure d'écart (40) disposé sur l'endoscope (16) ou intégré dans ce dernier, et
- au moins une image endoscopique de la surface (14) de la cavité du corps (12) dans la zone du ou des points sur la surface (14) de la cavité du corps (12),
sont associés à la ou aux positions et orientations,
dans lequel, à partir desdites données relatives à plusieurs positions et orientations différentes de l'endoscope (16), ledit ou lesdits extraits du modèle 3D est ou sont créés superposés à l'image endoscopique correspondante.

2. Procédé selon la revendication 1, dans lequel les aires (60, 62) de la surface (14) de la cavité du corps (12), desquelles une image endoscopique a déjà été déterminée, sont marquées visuellement sur le modèle virtuel de la cavité du corps (12).

3. Procédé selon la revendication 2, dans lequel le marquage des aires (60, 62) de la cavité du corps (12), pour lesquelles une image endoscopique a déjà été détectée, est effectué à l'aide de la détection de la durée passée à l'examen de ces aires, à l'aide d'une intensité lumineuse différente et/ou d'un signal qui est généré lors de l'examen d'une aire déterminée.

4. Procédé selon l'une quelconque des revendications 1 à 3, dans lequel ledit au moins un écart entre l'endoscope (16) et ledit au moins un point (42, 42') sur la surface (14) de la cavité du corps (12) est détecté au moyen d'au moins un faisceau laser émis par l'endoscope (16) par exemple de manière stéréoscopique sur la base d'une triangulation ou par une mesure du temps de propagation du faisceau laser.

5. Procédé selon l'une quelconque des revendications 1 à 4, dans lequel ledit au moins un écart entre l'endoscope (16) et ledit au moins un point (42, 42') sur la surface (14) de la cavité du corps (12) est détecté au moyen d'un motif projeté par l'endoscope (16) sur la surface (14) de la cavité du corps (12).

6. Procédé selon l'une quelconque des revendications 1 à 5, dans lequel ledit au moins un écart entre l'endoscope (16) et ledit au moins un point (42, 42') sur la surface (14) de la cavité du corps (12) est détecté au moyen d'ultrasons émis par l'endoscope (16) par le biais d'une mesure du temps de propagation des ultrasons.

7. Procédé selon l'une quelconque des revendications 1 à 6, dans lequel la mesure d'écart est exécutée à des intervalles de temps qui sont inférieurs à une seconde, qui sont de préférence dans la plage d'une milliseconde ou moins.

8. Procédé selon l'une quelconque des revendications 1 à 7, dans lequel ledit au moins un extrait du modèle virtuel de la cavité du corps (12) est superposé à ladite au moins une image endoscopique avec une image de la cavité du corps (12) obtenue par un procédé d'imagerie avec effet de profondeur, par exemple par tomographie assistée par ordinateur, résonance magnétique et/ou ultrasons.

9. Procédé selon l'une quelconque des revendications 1 à 8, dans lequel le modèle virtuel de la surface (14) de la cavité du corps (12) est déduit d'images endoscopiques qui sont obtenues d'abord partiellement par une excitation fluorescente.

10. Procédé selon la revendication 9, dans lequel seules les aires, qui présentent des anomalies spécifiques des tissus, sont affichées par excitation fluorescente dans le modèle virtuel.

11. Procédé selon l'une quelconque des revendications 1 à 10, dans lequel le modèle virtuel est converti par calcul d'une représentation 3D en une représentation 2D.

12. Dispositif de création d'abord d'un extrait d'un modèle 3D d'une cavité du corps (12), en particulier d'un organe creux, conformément à un procédé selon l'une quelconque des revendications 1 à 11, pourvu d'au moins endoscope (16) servant à la détection d'au moins une image endoscopique d'au moins une aire d'une surface (14) de la cavité du corps (12), d'un système de détection de position (30) servant à la détection de la position et de l'orientation de l'endoscope (16), **caractérisé par** un système de mesure d'écart (40) servant à la détection d'au moins un écart de l'endoscope (16) en au moins un point (42, 42') de la surface (14) de la cavité du corps (12) en fonction de la position et de l'orientation de l'endoscope (16), dans lequel le système de mesure d'écart (40) est disposé sur l'endoscope (16) ou intégré à ce dernier, et par un système de traitement de données (46), qui est conçu de sorte que, à partir d'une pluralité de points (42, 42') sur la surface (14) de la cavité du corps (12) détectés par le système de mesure d'écart (40) à partir de différentes positions et orientations de l'endoscope (16), il crée ledit ou lesdits extraits du modèle virtuel de la surface (14) de la cavité du corps (12) et projette ladite au moins une image endoscopique sur le modèle créé de cette façon.

13. Dispositif selon la revendication 12, **caractérisé en ce que** le système de traitement de données (46) est conçu de sorte qu'il marque visuellement sur le modèle virtuel de la cavité du corps (12) les aires desquelles une image endoscopique a déjà été détectée.

14. Dispositif selon la revendication 12 ou 13, dans lequel le système de traitement de données (46) est conçu de sorte qu'il déduise un autre modèle 3D virtuel de la cavité du corps (12) à partir d'une image de la cavité du corps obtenue par un procédé d'imagerie avec effet de profondeur, par exemple par tomographie assistée par ordinateur, par résonance magnétique, par ultrasons.

15. Dispositif selon la revendication 14, dans lequel le système de traitement de données (46) est conçu de sorte que le modèle virtuel, qui est déduit des informations d'image endoscopiques, soit superposé au modèle virtuel qui est obtenu à partir des informations du procédé d'imagerie avec effet de profondeur.

16. Dispositif selon la revendication 14 ou 15, dans lequel le système de traitement de données 50 est conçu de sorte à permettre un mode d'examen dans lequel, dans la direction de vision de l'endoscope (16), on puisse examiner davantage, en partant de l'image endoscopique de la surface (14) de la cavité du corps (12), les informations 3D correctement positionnées du procédé d'imagerie avec effet de profondeur.

17. Dispositif selon l'une quelconque des revendications 12 à 16, dans lequel le système de détection de position (30) comporte un système de capteur inertiel fixé à l'endoscope (32).

18. Dispositif selon l'une quelconque des revendications 12 à 17, dans lequel le système de mesure d'écart (40) est un système de mesure d'écart sans contact.
